# FASCICULE DE BREVET EUROPEEN

(11) **EP 3 270 887 B1**
(45) Date de publication et mention de la délivrance du brevet: **21.10.2020**
(21) Numéro de dépôt: 16713345.3
(22) Date de dépôt: 18.03.2016
(51) Int. Cl.: A61K 8/97, A23L 33/105, A61K 36/00, A23P 30/20, A61K 8/9711, A61K 8/9717, A61K 8/9722, A61K 8/9728, A61K 8/9733, A61K 8/9789, A61K 8/9794, A61K 36/185, A61K 36/28, A61K 36/61

(54) **PROCEDE DE PREPARATION D'UN EXTRAIT DE MATRICE VEGETALE PAR EXTRUSION AVEC UNE SOLUTION HYDROTROPIQUE**
VERFAHREN ZUR HERSTELLUNG EINES EXTRAKTES EINER MATRIX PFLANZLICHEN URSPRUNGS DURCH EXTRUSION MIT EINER HYDROTROPEN LÖSUNG
METHOD FOR PRODUCING AN EXTRACT OF A MATRIX OF VEGETABLE ORIGIN BY EXTRUSION WITH A HYDROTROPE SOLUTION

(30) Priorité: 18.03.2015 FR 1552249
(43) Date de publication de la demande: 24.01.2018
(73) Titulaire: Pierre Fabre Dermo-Cosmétique, 92100 Boulogne-Billancourt (FR)
(72) Inventeur: MANDEAU, Anne, 31200 Toulouse (FR); LETI, Mathieu, 31450 Montgiscard (FR)
(74) Mandataire: Regimbeau
(86) Numéro de dépôt international: PCT/EP2016/056039
(87) Numéro de publication internationale: WO 2016/146838

(56) Documents cités:
- EP-A1- 1 452 095
- JP-A- 2009 203 229
- KR-B1- 100 810 533
- US-A1- 2008 138 489
- US-B1- 6 365 601

## Description

Le domaine de la présente invention concerne un procédé de préparation d'un extrait de matrice végétale par extrusion avec une solution hydrotropique.

L'extrusion est un procédé par lequel un matériau susceptible de s'écouler sous diverses conditions contrôlées est ensuite contraint à passer dans une filière à vitesse déterminée (Dziezak, J. D. (1989). Single and twin-screw extruders in food processing. Food Technol., April, 164- 174). Initialement, cette technologie a été utilisée dans l'industrie métallurgique, en Angleterre, à la fin du 18ième siècle. Quelques temps plus tard, elle a été mise en place dans l'industrie agro-alimentaire pour la fabrication de saucisson, et de pâtes alimentaires. Aujourd'hui, l'industrie alimentaire utilise abondamment cette technique d'extrusion à travers la cuisson-extrusion des produits amylacés (biscuits, biscottes, snacks, etc.) mais aussi la texturation des protéines et la fabrication d'aliments pour les animaux d'élevage ou de compagnie.

Parallèlement, la technologie d'extrusion a été largement développée pour l'industrie des thermoplastiques, et a conduit à la conception de nouvelles vis, un développement de la technologie et une ouverture vers de nouvelles applications.

Plusieurs études ont ainsi porté sur l'utilisation de l'extrudeur pour conduire des actions chimiques, mécaniques, thermomécaniques en une seule étape et en continu, comme par exemple l'extraction d'hémicelluloses (N'Diaye, S., Rigal, L., Larocque, P., Vidal, P.F., 1996. Extraction of hemicelluloses from poplar populus tremuloides, using an extruder type twin-screw reactor: A feasibility study. Bioresearch Technology 57, 61-67), de pectines (Marechal V., Rigal L. Characterization of by-products of sunflower culture - Commercial applications for stalks and heads (1999) Industrial Crops and Products, 10 (3), pp. 185-200). etc.. Dans ces cas, un solvant acide ou basique est introduit dans l'extrudeur en même temps que la matière première végétale, afin de faciliter l'extraction et la solubilisation des macromolécules recherchées (extrusion réactive).

Certaines applications sont déjà connues dans l'extraction des végétaux : la mise en oeuvre de l'extrudeur mono-vis pour l'expression d'huiles à partir de graines oléagineuses avec lequel aucun solvant n'est injecté dans le fourreau, cette extraction d'huile reposant sur la compression du solide (Sriti J., Talou T., Faye M., Vilarem G. and Marzouk B. Oil extraction from coriander fruits by extrusion and comparison with solvent extraction processes. (2011) Industrial Crops and Products, 33, 659-664).

L'extrusion est également utilisée comme prétraitement, sur des marcs de fruits (pomme, cassis, canneberge..) en association avec un support solide tel que l'amidon de maïs, afin d'augmenter l'extraction des composés phénoliques (White Brittany L., Howard Luke L., Prior Ronald L.. Polyphenolic composition and antioxidant capacity of extruded cranberry pomace. (2010), J. Agric. Food Chem. 58, 4037-4042.).

US 2008/0138489 A1 divulgue un procédé d'obtention d'un extrait de thé, caractérisé en ce que les feuilles de thé sont soumises à une extraction dans un extrudeur bi-vis, suivie d'une extraction avec un solvant alcoolique.

Dans le domaine de l'extraction végétale, en fonction du solvant utilisé, les membranes des cellules végétales sont plus ou moins fragilisées, ce qui permet ou non la libération des composés qu'elles renferment.

L'eau est certes un solvant naturel considéré comme renouvelable ; mais sa forte polarité ne permet pas d'extraire certaines molécules lipophiles d'intérêt.

Les agents hydrotropes sont des composés organiques solubles dans l'eau qui, à partir d'une certaine concentration appelée « CMH » (pour « Concentration Minimale Hydrotropique »), permettent une augmentation significative de la solubilité de composés organiques pratiquement insolubles dans l'eau dans les conditions normales. Les hydrotropes sont amphiphiles et peuvent être ioniques (anioniques, cationiques, zwitterioniques) ou non ioniques (résorcinol, nicotinamide, alkyl polyglycosides etc.) et peuvent avoir diverses structures, par exemple aromatiques, aliphatiques, cycliques ou non. Les agents hydrotropes sont des composés solubles dans l'eau dans toute proportion et n'ayant pas de propriété tensioactive.

La concentration minimale hydrotropique (CMH) est la concentration à partir de laquelle les hydrotropes commencent à former des agrégats, c'est-à-dire de nouveaux micro-environnements avec des propriétés physiques différentes de celles observées lorsque le composé est dilué, et différentes d'un comportement micellaire. Cette concentration minimale hydrotropique est spécifique à chaque hydrotrope et est généralement de l'ordre de grandeur de la molarité. Elle peut être déterminée par plusieurs méthodes physico-chimiques, telles que la mesure de la tension de surface, la conductivité, ou la diffusion dynamique et statique de la lumière (Self-association of Nicotinamide in aqueous solution: Light-scattering and vapor pressure osmometry studies (1996) 85(8) : 848-853).

La CMH peut être déterminée par plusieurs méthodes physico-chimiques, telles que la mesure de la tension de surface, la conductivité, la diffusion dynamique et statique de la lumière (Self-association of Nicotinamide in aqueous solution: Light-scattering and vapor pressure osmometry studies (1996) 85(8): 848-853) ou tout simplement en établissant une courbe de solubilisation d'un composé lipophile (teneur en soluté solubilisé vs concentration en hydrotrope). Le rouge Soudan, colorant lipophile facilement dosable par spectrophotométrie, peut être utilisé comme référence. La valeur de cette concentration est dépendante de la nature de l'hydrotrope mais non du soluté. Elle correspond à la concentration minimale à partir de laquelle la courbe de solubilisation du soluté devient exponentielle.

Le potentiel des hydrotropes comme adjuvants de solubilisation en milieu aqueux de certaines molécules lipophiles a été exploré. (Da Silva R.C., Spitzer M., Da Silva L.H.M., Loh W. Investigations on the mechanism of aqueous solubility increase caused by some hydrotropes. (1999) Thermochimica Acta 328, 161-167) Le potentiel des hydrotropes ioniques comme adjuvants d'extraction de métabolites d'origine végétale en milieux aqueux a également été montré. (Dandekar D.V., Jayaprakasha G.K., Patil B.S., Hydrotropic extraction of bioactive limonin from sour orange (Citrus aurantium L.) seeds. (2008) Food Chemistry, 109, 515-520).

Il existe toujours un besoin d'améliorer les procédés d'extraction existants afin, entre autres, de diminuer leur impact environnemental et d'améliorer la qualité de l'extrait obtenu.

La Demanderesse a mis en évidence qu'un procédé de préparation d'un extrait de matrice végétale par extrusion avec une solution hydrotropique est particulièrement avantageux car il permet d'obtenir des extraits riches en composés d'intérêt et dans de plus larges gammes de polarités ; tout en réduisant la consommation énergétique. Avantageusement, le procédé offre également la possibilité d'utiliser des solvants alternatifs aux solvants traditionnels issus de la pétrochimie et qui sont à la fois plus respectueux de l'environnement et conduisent à des extraits particulièrement adaptés aux usages pharmaceutiques, cosmétiques ou agro-alimentaires.

La présente invention concerne un procédé d'extraction de matrices végétales sèches ou fraîches par extrusion en présence d'une solution aqueuse hydrotropique contenant au moins un agent hydrotrope de nature non ionique, en quantité suffisante pour extraire des composés lipophiles. L'extrusion permet d'obtenir un extrait total en très peu de temps, avec un rapport poids / solvant bien inférieur à une extraction classique, et une qualité comparable voire supérieure. L'impact environnemental du procédé est ainsi largement amélioré. De plus, dans le cadre de la présente invention, l'extrait peut être soit total (contenant des composés polaires, moyennement polaires et lipophiles), soit enrichi en composés lipophiles d'intérêt du fait de la sélectivité de l'extraction à l'aide de la solution hydrotropique.

Plus précisément, l'invention vise un procédé d'obtention d'un extrait de matrice végétale, caractérisé en ce que lesdites matrices végétales fraîches ou sèches sont soumises à un traitement mécanique consistant à extruder la matrice dans un extrudeur, associé ou non à un traitement thermique, en présence d'une solution aqueuse contenant au moins un agent hydrotrope de préférence à une concentration minimale hydrotropique, suivi d'une opération de récupération de l'extrait.

Par solution aqueuse contenant au moins un agent hydrotrope à une concentration au moins égale à la concentration minimale hydrotropique (CMH), on entend selon la présente invention une solution aqueuse contenant au moins un agent hydrotrope à une concentration supérieure ou égale à la concentration minimale hydrotropique (CMH) précédemment rappelée. Il faut également tenir compte de la teneur en eau éventuellement présente dans la matrice végétale et ajuster en conséquence la concentration des agents hydrotropes pour permettre leur bonne utilisation dans le procédé selon la présente invention.

Selon une autre caractéristique de l'invention, la concentration de l'agent hydrotrope dans ladite solution aqueuse est comprise entre 1 à 10 fois, de préférence entre 1 à 6 fois, de préférence encore entre 1 à 2 fois, de manière encore plus préférentielle entre 1,4 à 1,8 fois de la concentration minimale hydrotropique (CMH). Avantageusement, dans la pratique on pourra utiliser l'agent hydrotrope dans une solution aqueuse à une concentration égale à 1,5 mol/L.

Selon une autre caractéristique avantageuse de la présente invention, l'agent hydrotrope est présent dans la solution aqueuse d'extraction à une concentration inférieure à 60 % en poids par rapport au poids de ladite solution, de préférence inférieure à 50 % en poids par rapport au poids de ladite solution, de préférence encore inférieure à 40 % en poids par rapport au poids de ladite solution de manière encore plus préférentielle inférieure à 30 % en poids par rapport au poids de ladite solution. On observera en particulier que ce seuil de concentration écarte implicitement l'utilisation de l'éthanol comme solvant d'extraction de composés lipophiles dans la mesure où l'éthanol est en général utilisé à des teneurs très supérieures, de l'ordre de 80 %. De plus l'utilisation d'éthanol dans un extrudeur impliquerait nécessairement un environnement sécurisé en raison des risques encourus consécutifs à la volatilité et l'inflammabilité de ce solvant particulier.

Par « composés lipophiles », on entend au sens de la présente invention, des composés avec un coefficient de partage octanol/eau, encore appelé logP ou log kow, positif.

Selon l'invention, l'agent hydrotrope non ionique, préférentiellement agrosourcé, est choisi parmi les alkyl-(poly)glycosides de formule générale Alk-O-Zp, dans laquelle :
- Alk désigne un fragment hydrophobe aliphatique hydrocarboné, saturé ou insaturé, linéaire ou ramifié comprenant de 3 à 7 atomes de carbone, et
- Z représente un groupement glycoside hydrophile tel que le glucose, le xylose et l'arabinose, et
- 1 < p < 5.

Selon un mode de réalisation particulier de l'invention, Z représente un groupement glucose.

Selon un autre mode de réalisation particulier de l'invention, Z représente un groupement xylose.

Selon encore un autre mode de réalisation particulier de l'invention, Z représente un groupement arabinose.

Selon un mode de réalisation particulier de l'invention, Alk désigne un fragment hydrophobe aliphatique hydrocarboné, saturé ou insaturé, linéaire ou ramifié comprenant 7 atomes de carbone.

Selon un autre mode de réalisation particulier de l'invention, Alk désigne un fragment hydrophobe aliphatique hydrocarboné, saturé ou insaturé, linéaire ou ramifié comprenant 6 atomes de carbone.

Selon encore un autre mode de réalisation particulier de l'invention, Alk désigne un fragment hydrophobe aliphatique hydrocarboné, saturé ou insaturé, linéaire ou ramifié comprenant 5 atomes de carbone.

Selon encore un autre mode de réalisation particulier de l'invention, Alk désigne un fragment hydrophobe aliphatique hydrocarboné, saturé ou insaturé, linéaire ou ramifié comprenant 4 atomes de carbone.

Dans un autre mode de réalisation de l'invention, l'agent hydrotrope est agrosourcé (d'origine non pétrochimique).

Selon une autre caractéristique de l'invention, l'agent hydrotrope agrosourcé est une combinaison d'un alcool gras en C7 dérivé de ricin avec du glucose de blé (sans OGM).

Selon une caractéristique avantageuse de l'invention, ledit agent hydrotrope est un amyl-glycoside dont le fragment hydrophobe amyl correspond à un alcool en C5 obtenu par fermentation de la betterave ou de la fécule de pomme de terre et dont le fragment glycoside provient de céréales.

Selon une caractéristique avantageuse de l'invention, ledit agent hydrotrope est une combinaison d'un alcool gras en C4 avec un xyloside.

Dans un autre mode de réalisation particulier de l'invention, ledit agent hydrotrope de préférence agrosourcé, est un diol en particulier l'isopentyldiol (3-Methyl-1,3-butanediol).

Selon une caractéristique de l'invention, l'agent hydrotrope est de type non ionique, en particulier choisi parmi l'isopentyldiol, l'heptylglucoside et le butylxyloside.

Le composé amphiphile non ionique, utile dans le cadre de la présente invention, peut avantageusement être une des matières premières commerciales SEPICLEAR G7® (Société SEPPIC), APPYCLEAN® (Société WHEATOLEO), Isopentyldiol de Kuraray.

Selon une autre caractéristique de l'invention, la matrice végétale subit un pré-traitement avant l'étape d'extrusion. Par « pré-traitement », on entend selon la présente invention un traitement choisi parmi les traitements suivants : aux ultra-sons, par micro-ondes, une digestion enzymatique, une macération dans la solution hydrotropique, une préparation de lamatrice végétale telle que cryobroyage, fragmentation, broyage, etc.

Dans un mode de réalisation particulier de l'invention, le pré-traitement consiste en une macération dans la solution hydrotropique avant l'étape d'extrusion.

Par «extrusion» on entend selon la présente invention un traitement mécanique consistant à extruder la matrice végétale dans un extrudeur monovis ou bivis, à vis co- ou contra-rotatives, de préférence un extrudeur bi-vis, associé ou non à un traitement thermique.

L'extrusion est réalisée à des températures comprises entre 20 et 200°C, préférentiellement entre 60 et 120°C.

Selon une autre caractéristique de l'invention, l'extrusion est mise en oeuvre dans un extrudeur bi-vis.

Selon une caractéristique avantageuse de l'invention, l'extrudeur bi-vis comporte une première zone bi-vis corotatives et copénétrantes où se réalise la trituration desdites matrices végétales.

Le procédé met en oeuvre un extrudeur et de préférence un extrudeur bi-vis, à plusieurs fourreaux et terminé par un fourreau filtrant, permettant de faire varier la température et en même temps d'appliquer un cisaillement, un malaxage intense de la matière première végétale, avec pour conséquence d'entraîner un grand nombre de composés, de déstructurer la matière.

L'extrudeur est alimenté par la matrice végétale et la solution hydrotropique, avec des débits déterminés de façon à obtenir des rapports poids de matrice végétale en kilo / volume de solvant en L allant de 1P/1V à 1P/10V, préférentiellement de 1P/2V à 1P/6V.

La présente invention concerne également l'utilisation d'une solution aqueuse contenant au moins un agent hydrotrope à une concentration minimale hydrotropique dans un procédé d'extraction de matrices végétales fraîches ou sèches, mis en oeuvre par traitement mécanique conduit dans un extrudeur. Les conditions d'extrusion ainsi que la nature des matrices végétales utilisées sont identiques à celles décrites à propos du procédé selon l'invention.

Par « matrice végétale », » on entend selon la présente invention tout ou partie de plante, de champignon, de lichen, d'algue, de culture de micro-algues ou de culture de cellules végétales dédifférenciées.

Lesdites plante, champignon, lichen et algue sont sèches ou fraîches, congelées ou décongelées et entières (non fragmentées et non broyées), fragmentées ou broyées. Lesdites cultures de micro-algues ou de cellules végétales dédifférenciées sont entières, broyées, fraiches de préférence ou séchées, filtrées pour récupérer la biomasse, ont ou non subit un pré-traitement afin de libérer le contenu intra-cellulaire, par exemple par un procédé ultrasonique.

Par « partie de plantes », on entendra notamment les parties aériennes telles que tiges, branches, feuilles, fruits, graines et/ou fleurs ; et/ou les parties souterraines telles que les rhizomes, les racines et/ou les bulbes.

Par « partie de lichen, de champignon ou d'algue », on entendra tout organe de ces organismes tels que les thalles, les sporophores, les macromycètes, les mycéliums, et/ou les filaments.

Dans un mode de mise en œuvre particulier de l'invention, on utilisera tout ou partie de plantes entières (non fragmentée, non broyée)

Parmi les plantes utilisables dans le cadre de la présente invention, on peut citer entre autres : les fruits de *Physalis peruviana*, les fruits *d'Embelia ribes*, les feuilles de *Myrtus communis,* les feuilles d*'Eucalyptus globulus,* les péricarpes de *Garcinia mangostana,* les inflorescences femelles d*'Humulus lupulus*, l'écorce de *Cinchona* sp., les parties aériennes d'*Urtica dioica*, les parties aériennes d'*Helichrysum* spp., les fruits de *Vanilla* spp., les rhizomes de *Zingiber officinale,* les rhizomes de *Curcuma* spp., les rhizomes *de Piper methysticum*, les feuilles de *Piper* spp. , les fruits et feuilles d'*Olea europaea...*

Parmi les algues utilisables dans le cadre de la présente invention, on entend en particulier les algues bleues ou Cyanobactéries ainsi que des eucaryotes parmi lesquels les Euglénophytes, Cryptophytes, Haptophytes, Gaucophytes, les algues rouges ou *Rhodophyta,* les *Stramenopiles* regroupant notamment les Diatomées et les algues brunes ou Phéophycées, et enfin les algues vertes qui comprennent entre autres les Ulvophycées.

Parmi les lichens utilisables dans le cadre de la présente invention, on peut citer en autres : les thalles de *Cetraria islandica,* les thalles de *Usnea* spp., les thalles de *Cladonia* spp., les thalles de *Lobaria* spp.

Parmi les champignons utilisables dans le cadre de la présente invention, on peut citer entre autres *Coriolus versicolor*, *Cordyceps* spp.

Parmi les cultures de cellules végétales utilisables dans la présente invention, on peut citer entre autre : les cultures de cellules de *Mimosa pudica*, *Tripterygium wilfordii.*

Dans un mode de réalisation, l'extrusion se caractérise par le passage de la matrice végétale sèche ou fraîche dans un extrudeur bivis composé de :
- une zone d'introduction: Trémie d'alimentation
- le corps principal de l'extrudeuse est constitué d'un ou plusieurs fourreaux dans lesquels tournent les vis sans fin (corotatives ou contrarotatives), ou segments de vis. De préférence, il s'agira de plusieurs fourreaux successifs adjacents. De préférence, il s'agira de deux vis sans fin corotatives. Le profil des vis pouvant varier selon la forme du filet des vis (par ex trapézoïdal, conjugué, simple ou double...) et du pas de vis. Chacune de ces vis peut également présenter différents tronçons (ou segments) qui peuvent éventuellement différer les uns des autres, par la forme du filet et/ou par le pas de vis. Eventuellement, certains des tronçons constitutifs de ces vis peuvent également correspondre à des éléments malaxeurs monolobes, ou trilobes ;
- un moyen d'alimentation en solution aqueuse d'agent hydrotrope remplissant la fonction de solvant, ledit moyen d'alimentation étant couplé à au moins un des fourreaux;
- au moins un fourreau filtrant qui :
   ✔ intervient le cas échéant pour la séparation solide/liquide ;
   ✔ comprend en outre un moyen de filtration, tel qu'une grille, et ;
   ✔ se trouve en particulier situé à la sortie de l'extrudeur ;
- des moyens de chauffage et de refroidissement car le fourreau doit être régulé en température : de 20°C à 200°C.
- des moyens de pilotage de l'extrudeur tels que :
   ✔ un groupe d'entraînement : composé d'un moteur réducteur et d'un diviseur de couple, qui fournissent la puissance mécanique nécessaire à la rotation des vis ;
   ✔ automates de pilotage : permettent le suivi et la commande du procédé. Les paramètres pouvant être réglés sont : la vitesse de rotation des vis et la température de chaque fourreau.

Le procédé mécanique d'extrusion bi-vis entraîne la formation d'un bouchon végétal apportant une pression sur la matière ainsi qu'un éclatement des cellules, une déstructuration de la matière végétale permettant d'extraire un maximum de composés qui seront entrainés et solubilisés dans la solution hydrotropique.

La collecte de l'extrait consistant à séparer le solvant chargé en composés d'intérêt des résidus solides résiduels provenant de la matrice végétale peut ensuite se faire par clarification et/ou filtration.

Par « clarification » on entend élimination des fragments de cellules présents dans l'extrait à la sortie de l'extrudeuse. Cette élimination peut se faire grâce à la technologie de clarification par effet centrifuge, qui a pour but d'éliminer le résidu solide qui pourrait colmater le média filtrant. Cette élimination peut aussi se faire directement par filtration avec un adjuvant.

Par « filtration » on entend filtration frontale ou tangentielle, où l'on peut envisager la présence d'adjuvant de filtration (type perlite, diatomées, etc..). Cette filtration permet de retenir les derniers résidus solides, le but étant d'obtenir une solution parfaitement limpide. Elle peut être suivie d'une filtration sur membrane avec un seuil de coupure défini en fonction de la taille des molécules à considérer. Elle peut également être remplacée ou suivie par une filtration sur résine ou silice, afin d'enrichir en composé d'intérêt (ex : résines d'adsorption).

Dans un mode de réalisation particulier, l'étape de clarification - filtration sera réalisée à l'aide d'un fourreau filtrant intégré en fin d'extrudeuse.

Dans un mode réalisation particulier, l'extrait est conservé comme tel ou lyophilisé, comprenant les molécules d'intérêt, ainsi que le ou les hydrotropes, ces derniers permettant une meilleure solubilisation dans le produit fini.

On obtient ainsi un extrait total contenant des composés de polarité large (polaire, moyennement polaire, apolaire).

L'extrait peut également être dilué dans un volume d'eau additionnée ou non d'un ou plusieurs adjuvants choisis parmi des sels, acides ou bases, de façon à se situer à une concentration finale en agent hydrotropique inférieure à la CMH. Ainsi, il est possible de récupérer les composés lipophiles par précipitation et séparation solide / liquide telle que la filtration ou centrifugation.

On obtient ainsi un extrait enrichi en composés lipophiles.

Les composés lipophiles d'intérêt peuvent être des flavonoïdes, acides phénoliques, composés terpéniques (mono-, di-, triterpénes) et stéroïdiques, des dérivés diarylheptanoïdes, des lignanes, coumarines, quinones, anthraquinones, xanthones, phloroglucinols, iridoïdes, lactones sesquiterpéniques, des alcaloïdes, des sucroesters, des lipides polaires...

Ils peuvent être en particulier les kavalactones, myrtucommulones, l'embeline, la quinine et ses dérivés, la vanilline et ses dérivés, l'a-mangostine, le xanthohumol, les mono et digalactosyldiacylglycérols, , l'acide maslinique, l'acide ursolique, l'acide rosmarinique, le carnosol, la galangine, pinobanksine, cardamonine, les curcuminoïdes, le gingérol, shogaol,...

L'extrait total ou l'extrait enrichi en composés lipophiles peuvent être dilués, concentrés, séchés ou conservés comme tel, par l'ajout de conservateur adapté et autorisé dans le produit fini souhaité (tels que les glycols, ou l'acide sorbique, l'acide benzoïque, l'acide citrique et leurs sels, etc) ou d'alcool (minimum 15°).

Pour la fourniture d'un extrait sec, les technologies de séchage sous vide, de lyophilisation ou d'atomisation peuvent être envisagées. L'extrait obtenu peut être séché avec ou sans support et / ou solubilisé dans un support liquide.

Les extraits obtenus, liquides, pâteux ou secs tels que définis plus haut peuvent être utilisés en l'état dans des compositions cosmétiques, pharmaceutiques ou alimentaires, destinées à être administrées par voie topique ou voie orale.

Les avantages de l'extraction hydrotropique par extrusion en comparaison d'une extraction en réacteur sont :
- la diminution significative de la quantité de solvant mise en oeuvre (de 2 à 5 fois inférieur en moyenne)
- la réduction significative du temps d'extraction (2 à 5 minutes contre 1 heure au moins)
- la possibilité d'utilisation d'un solvant alternatif agrosourcé (tel que les polyalkyl glycosides notamment) à la place de solvants d'origine pétrochimique polluants (acétate d'éthyle, acétone, hexane...)
- la qualité de l'extrait obtenu (extrait de polarité large ou extrait enrichi en composés lipophiles d'intérêt ; et sélectivité également observée parmi les composés lipophiles d'intérêt)

Selon un mode de réalisation préféré, outre la solution hydrotropique, aucun autre solvant n'intervient dans l'étape d'extrusion proprement dite. La solution hydrotropique est l'unique solvant intervenant dans le procédé d'extraction par extrusion.

Les exemples suivants sont donnés à titre indicatif non limitatif.

### EXEMPLES

### Exemple 1 : Extrait de Myrte

1,3 kg de feuilles séchées de *Myrtus communis* sont introduits dans le premier fourreau d'un extrudeur bivis Clextral BC45 avec un débit de 8 kg/H. Est ensuite introduite une solution aqueuse d'amyl xylosides à 1,5 mol/L avec un débit de 24L/H. La température appliquée aux différents fourreaux est de 60°C/60°C/60°C/60°C/60°C. Au bout de 5 minutes, l'extrait de feuilles de Myrte est récupéré en sortie d'extrudeur grâce à un fourreau filtrant permettant une séparation solide / liquide. Après clarification, l'extrait sec de Myrte est obtenu avec un rendement de 81% par rapport au solvant engagé et 1184% par rapport à la plante engagée. La solution est diluée par 4 volumes d'eau. Après centrifugation, le culot correspondant à l'extrait enrichi de Myrte est obtenu avec un rendement massique de 1,2% par rapport à la plante engagée.

Pour comparaison, 1,3 kg de feuilles séchées de *Myrtus communis* sont extraites par de l'eau dans les mêmes conditions. Après clarification, l'extrait de Myrte est obtenu avec un rendement de 8,5% par rapport à la plante engagée.

Les myrtucommulones B', S et A sont dosées dans les 2 extraits ainsi que dans deux extraits réalisés en réacteur.

| **Extrait** | **Durée extraction** | **Rendement** | **Teneur en myrtucommulones dans l'extrait (m/m)** | **Rapport [Myrt. B'] / [Myrt. A]** |
|---|---|---|---|---|
| Amyl xyloside 1P/3V extrusion + précipitation par dilution | 5' | 1,2% | 9,9 % | 2,3 |
| Amyl xyloside 1P/7V réacteur + précipitation par dilution | 3H | 2,1% | 8,9% | 1,9 |
| Acetate d'isopropyle 1P/8V réacteur | 2H | 3,1% | 7,3% | 3,1 |
| Eau 1P/3V extrusion | 5' | 8,5% | 0% | / |

Bien que le rendement massique d'extraction soit plus faible, l'extrait obtenu par extrusion, avec un rapport poids plante / volume solvant irréalisable en réacteur (non suffisant pour mouiller la plante), et une durée d'extraction extrêmement rapide, est un peu plus concentré en myrtucommulones que celui obtenu par une extraction classique en réacteur avec le même solvant. Il est également plus concentré en myrtucommulones que l'extrait obtenu par acétate d'isopropyle en réacteur. La myrtucommulone A, la plus apolaire, est deux fois plus riche dans l'extrait hydrotropique obtenu par extrusion que dans l'extrait par acétate d'isopropyle, ce qui met en évidence la sélectivité du procédé. L'extrait obtenu par extrusion avec de l'eau sans amyl xylosides ne permet pas d'extraire les composés d'intérêt.

### Exemple 2 : Extrait d'Helichrysum gymnocephalum par extrusion

5 kg de parties aériennes séchées entière d'Helichrysum gypmnocephalum sont introduits dans le premier fourreau d'un extrudeur bivis Clextral BC45 avec un débit de 10 kg/H. Est ensuite introduite une solution aqueuse contenant 50% d'heptylglucoside m/m (1,5M) avec un débit de 60L/H. La température appliquée aux différents fourreaux est de 60°C/60°C/60°C/60°C/60°C. Au bout de 5 minutes, l'extrait d'Helichrysum est récupéré en sortie d'extrudeur grâce à un fourreau filtrant permettant une séparation solide / liquide. Après clarification, l'extrait d'Helichrysum est obtenu avec un rendement de 69.5% par rapport au solvant engagé, et 407% par rapport à la plante engagée.

| **Extrait** | **Durée extraction** | **Rendement** | **Teneur en flavonoïdes apolaires dans l'extrait non concentré, non séché (m/m)** |
|---|---|---|---|
| Heptylglucoside 1P/6V extrusion | 5' | 407,1% | 0,13% |
| Heptylglucoside 1P/3V extrusion | 5' | 196,1% | 0,15% |
| Heptylglucoside 1 P/15V réacteur | 2H | 1450% | 0,10% |
| Eau 1P/6V extrusion | 5' | 573% | 0% |

Bien que les rendements d'obtention soient plus faibles, la qualité d'extrait obtenu par extrusion, avec des volumes de solvant jusqu'à 5 fois inférieurs, un rapport poids / solvant irréalisable en réacteur (non suffisant pour mouiller la plante), et une durée d'extraction extrêmement rapide, est comparable à celle obtenu par une extraction classique avec le même solvant. L'extrait obtenu par extrusion avec de l'eau sans heptylglucoside ne permet pas d'extraire ces composés d'intérêt.

### Exemple 3 : Extrait d'ortie sèche et d'ortie fraîche

0.67 kg de parties aériennes sèches d'ortie sont engagées dans le 1^{ier} fourreau d'un extrudeur bivis Clextral BC45 avec un débit de 8 kg/H. Est ensuite introduit une solution aqueuse contenant 10% d'heptylglucoside m/m avec un débit de 36 kg/H, ce qui correspond à une extraction avec un rapport poids/volume de 1/4,5, infaisable en batch au vu de la faible densité des parties aériennes d'ortie sèches. La température appliquée aux différents fourreaux est de 60°C/60°C/60°C/60°C/60°C. Au bout de 5 minutes, l'extrait d'ortie sèche est récupéré en sortie d'extrudeur grâce à un fourreau filtrant permettant une séparation solide / liquide. Après clarification, l'extrait d'ortie est obtenu avec un rendement de 48.7% par rapport au solvant engagé, et 218% par rapport à la plante engagée.

4.4 kg de parties aériennes fraîches d'ortie sont engagées dans le 1^{ier} fourreau d'un extrudeur bivis Clextral BC45 avec un débit de 33 kg/H. Est ensuite introduit une solution aqueuse contenant 10% d'heptylglucoside m/m avec un débit de 17 kg/H, ce qui correspond à une extraction avec un rapport poids/volume de 1/0.5, mais en réalité 1/6 si l'on tient compte de la teneur en eau de la plante. Ceci est impossible à réaliser en batch. La température appliquée aux différents fourreaux est de 60°C/60°C/60°C/60°C/60°C. Au bout de 8 minutes, l'extrait d'ortie fraîche est récupéré en sortie d'extrudeur grâce à un fourreau filtrant permettant une séparation solide / liquide. Après clarification, l'extrait d'ortie est obtenu avec un rendement de 162.6% par rapport au solvant engagé, et 85% par rapport à la plante engagée.

Les 2 extraits obtenus sont ensuite acidifié avec de l'acide sulfurique qs pH3 puis dilués 6 fois avec de l'eau, de manière à obtenir une concentration finale en heptylglucoside de 1.4%. Un trouble apparaît, et un culot est récupéré après centrifugation 20' à 3500 tr/min.

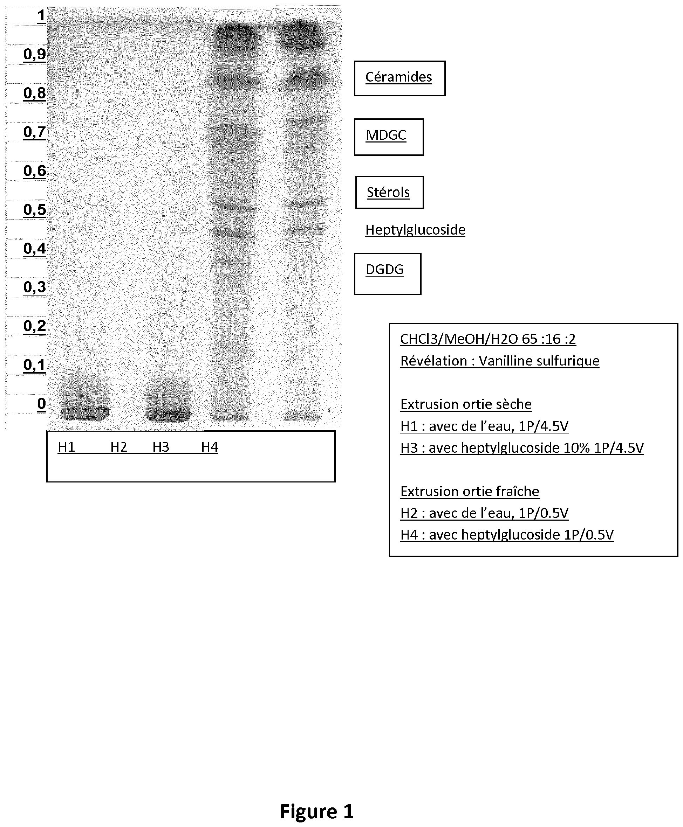

Une analyse par chromatographie CCM en figure 1 annexée montre dans ces 2 extraits la présence de lipides polaires comme les galactolipides et stérols, ces bandes étant absentes de l'extrait obtenu par extrusion en n'utilisant, dans les mêmes conditions (extrusion, plante sèche / fraîche, débits plante et solvant), que de l'eau. MGDG désigne le mono-galactosyldiacylglycérol et DGDG désigne le di-galactosyldiactylglycérol.

### Exemple 4 : gélule

| | |
|---|---|
| Extrait de Myrte selon l'exemple 1 | 200 mg |
| Amidon | 45 mg |
| Stéarate de magnésium | 2 mg |

### Exemple 5 : crème

| | |
|---|---|
| Extrait d'Helichrysum gymnocephalum selon ex 2 : | 0,5-3 % |
| Tribehenin PEG-20 esters | 2-7 % |
| Isodecyl neopentanoate | 2-9 % |
| Glycérine | 0.5-10 % |
| Glycol palmitate | 1-6 % |
| Cetyl alcohol | 0.5 - 3% |
| Disodium EDTA | 0.05-0.25 % |
| Conservateurs | 0.5-3 % |
| Parfum | 0.2-0.5 % |
| Xanthan gum | 0.1-0.4 % |
| Eau | qs |

## Revendications

1. Procédé d'obtention d'un extrait de matrice végétale, en particulier de plante, **caractérisé en ce que** la matrice végétale est soumise à un traitement mécanique consistant à extruder la matrice végétale dans un extrudeur, associé ou non à un traitement thermique, en présence d'une solution aqueuse contenant au moins un agent hydrotrope, à une concentration au moins égale à la concentration minimale hydrotropique, suivi d'une opération de récupération de l'extrait, **caractérisé en ce que** l'agent hydrotrope est un alkyl-(poly)glycoside de formule générale Alk-O-Zp, dans laquelle :
• Alk désigne un fragment hydrophobe aliphatique hydrocarboné, saturé ou insaturé, linéaire ou ramifié comprenant de 3 à 7 atomes de carbone, et
• Z représente un groupement glycoside hydrophile tel que le glucose, le xylose et l'arabinose, et
• 1 < p < 5.

2. Procédé selon la revendication 1, **caractérisé en ce que** ledit traitement thermique est conduit à des températures comprises entre 20°C et 200°C, de préférence entre 60°C et 120°C.

3. Procédé selon l'une des revendications 1 ou 2, **caractérisé en ce que** les plantes soumises au traitement thermomécanique en présence de solution aqueuse d'agent hydrotrope sont constituées par tout ou partie des parties aériennes et/ou des parties souterraines de plantes sèches ou de plantes fraîches, entières, fragmentées ou broyées.

4. Procédé selon la revendication 3 en ce que les parties de plantes sont représentées par les parties aériennes telles que tiges, branches, feuilles, fruits, graines et/ou fleurs ; et/ou les parties souterraines telles que les rhizomes, les racines et/ou les bulbes.

5. Procédé selon l'une des revendications 1 à 4, **caractérisé en ce que** les plantes sèches ou fraîches soumises au traitement thermomécanique en présence de solution aqueuse d'agent hydrotrope sont choisies parmi les fruits de *Physalis peruviana*, les graines d*'Embelia ribes*, les feuilles de *Myrtus communis,* les feuilles d'*Eucalyptus globulus*, les péricarpes de *Garcinia mangostana*, les inflorescences femelles d'*Humulus lupulus*, l'écorce de *Cinchona* sp., les parties aériennes d'*Urtica dioica*, les parties aériennes d'*Helichrysum* spp., les fruits de *Vanilla* spp., les rhizomes de *Zingiber officinale*, les rhizomes de *Curcuma* spp., les rhizomes de *Piper methysticum*, les feuilles de *Piper* spp, les fruits et feuilles d'*Olea europaea.*

6. Procédé selon l'une des revendications précédentes, **caractérisé en ce que** les plantes subissent un pré-traitement avant extrusion, un traitement aux ultra-sons, par micro-ondes, une digestion enzymatique, une macération dans la solution hydrotropique, une préparation de la plante telle que cryobroyage, fragmentation, broyage, en particulier une macération dans la solution hydrotropique.

7. Procédé selon l'une des revendications précédentes, **caractérisé en ce que** l'extrait récupéré est soumis à une étape ultérieure de clarification et/ou filtration.

8. Procédé selon les revendications 1 à 6 **caractérisé en ce que** l'extrait récupéré est dilué, concentré, séché ou conservé comme tel, par l'ajout de conservateur adapté.

9. Procédé selon les revendications 1 à 6 **caractérisé en ce que** l'extrait récupéré est dilué dans un volume d'eau suffisant pour récupérer un extrait sec enrichi en composés lipophiles.

10. Procédé selon l'une des revendications précédentes, **caractérisé en ce que** l'extrusion est mise en œuvre dans un extrudeur bi-vis.

11. Procédé selon la revendication 10, **caractérisé en ce que** l'extrudeur bi-vis comporte une première zone bi-vis corotatives et copénétrantes où se réalise la trituration desdites plantes.

12. Procédé selon l'une des revendications 10 et 11, **caractérisé en ce que** l'extrudeur bi-vis comprend une seconde zone bi-vis où se réalise la séparation solide/liquide.

13. Procédé selon l'une des revendications 1 à 12, **caractérisé en ce que** ledit extrudeur comporte au moins un fourreau et de préférence plusieurs fourreaux successifs adjacents.

## Patentansprüche

1. Verfahren zur Herstellung eines Pflanzenmatrixextrakts, insbesondere aus einer Pflanze, **dadurch gekennzeichnet, dass** die Pflanzenmatrix einer mechanischen Behandlung unterzogen wird, die darin besteht, die Pflanzenmatrix in einem Extruder, in oder ohne Verbindung mit einer Wärmebehandlung, zusammen mit einer wässrigen Lösung zu extrudieren, die zumindest ein hydrotropes Mittel in einer Konzentration enthält, die zumindest gleich der hydrotropen Mindestkonzentration ist, mit anschließender Rückgewinnung des Extrakts, **dadurch gekennzeichnet, dass** das hydrotrope Mittel ein Alkyl-(poly)glykosid der allgemeinen Formel Alk-O-Zp ist, in der:
• Alk ein hydrophobes, aliphatisches kohlenwasserstoffhaltiges, gesättigtes oder ungesättigtes, lineares oder verzweigtes Fragment bezeichnet, das 3 bis 7 Kohlenstoffatome umfasst, und
• Z für eine hydrophile Glykosidgruppe wie Glukose, Xylose und Arabinose steht, und
• 1<p<5.

2. Verfahren nach Anspruch 1, **dadurch gekennzeichnet, dass** die Wärmebehandlung bei Temperaturen zwischen 20°C und 200°C, vorzugsweise zwischen 60°C und 120°C, durchgeführt wird.

3. Verfahren nach einem der Ansprüche 1 oder 2, **dadurch gekennzeichnet, dass** die Pflanzen, die der thermomechanischen Behandlung zusammen mit einer wässrigen Lösung eines hydrotropen Mittels unterzogen werden, aus den gesamten oder einem Teil der oberirdischen und/oder unterirdischen Teile von trockenen oder frischen, ganzen, fragmentierten oder gemahlenen Pflanzen bestehen.

4. Verfahren nach Anspruch 3, wobei die Pflanzenteile durch die oberirdischen Teile wie Stängel, Zweige, Blätter, Früchte, Samen und/oder Blüten dargestellt werden; und/oder die unterirdischen Teile durch Rhizome, Wurzeln und/oder Zwiebeln dargestellt werden.

5. Verfahren nach einem der Ansprüche 1 bis 4, **dadurch gekennzeichnet, dass** die der thermomechanischen Behandlung zusammen mit einer wässrigen Lösung eines hydrotropen Mittels unterzogenen trockenen oder frischen Pflanzen aus den Früchten von *Physalis peruviana* (Kapstachelbeeren), den Samen von *Embelia ribes*, den Blättern von *Myrtus communis* (Myrtus), den Blättern von *Eucalyptus globulus*, den Perikarpen von *Garcinia mangostana* (Mangostanbaum), den Fruchtzapfen weiblicher *Humulus lupulus* (Hopfenpflanzen), der Rinde von *Cinchona* sp., den oberirdischen Teilen von *Urtica dioica* (Brennesse), den oberirdischen Teilen von *Helichrysum* spp., den Früchten von *Vanilla* spp. (Vanille), den Rhizomen von *Zingiber officinale,* den Rhizomen von *Curcuma* spp. (Curcuma SPP), den Rhizomen von *Piper methysticum* (Pfeffer), den Blättern von *Piper* spp (Pfeffer SPP), den Früchten und Blättern von *Olea europaea* (Olivenbaum) ausgewählt sind.

6. Verfahren nach einem der vorstehenden Ansprüche, **dadurch gekennzeichnet, dass** die Pflanzen vor der Extrusion einer Vorbehandlung, einer Ultraschallbehandlung, einer Mikrowellenbehandlung, einem enzymatischen Aufschluss, einer Mazeration in der hydrotropen Lösung, einer Vorbereitung der Pflanze wie Kryomahlung, Fragmentierung, Mahlung, insbesondere einer Mazeration in der hydrotropen Lösung unterzogen werden.

7. Verfahren nach einem der vorstehenden Ansprüche, **dadurch gekennzeichnet, dass** der gewonnene Extrakt einer weiteren Phase mit Klären und/oder Filtern unterzogen wird.

8. Verfahren nach der Ansprüche 1 bis 6, **dadurch gekennzeichnet, dass** der gewonnene Extrakt verdünnt, konzentriert, getrocknet oder als solcher durch Zugabe eines geeigneten Konservierungsmittels konserviert wird.

9. Verfahren nach der Ansprüche 1 bis 6, **dadurch gekennzeichnet, dass** der gewonnene Extrakt in einem Wasservolumen verdünnt wird, das ausreicht, um einen mit lipophilen Verbindungen angereicherten Trockenextrakt zu gewinnen.

10. Verfahren nach einem der vorstehenden Ansprüche, **dadurch gekennzeichnet, dass** die Extrusion in einem Zweischneckenextruder durchgeführt wird.

11. Verfahren nach Anspruch 10, **dadurch gekennzeichnet, dass** der Zweischneckenextruder einen ersten Bereich mit korotierenden und mitdurchdringenden Zweischnecken umfasst, in dem die Zerkleinerung der genannten Pflanzen durchgeführt wird.

12. Verfahren nach einem der Ansprüche 10 und 11, **dadurch gekennzeichnet, dass** der Zweischneckenextruder einen zweiten Zweischneckenbereich umfasst, in dem eine Fest-Flüssig-Trennung stattfindet.

13. Verfahren nach einem der Ansprüche 1 bis 12, **dadurch gekennzeichnet, dass** der Extruder zumindest ein Gehäuse und vorzugsweise mehrere aufeinanderfolgende anschließende Gehäuse umfasst.

## Claims

1. Method for obtaining an extract of a vegetable matrix, particularly plant matrix, **characterized in that** the vegetable matrix is subjected to mechanical treatment consisting of extruding the vegetable matrix in an extruder, associated or not with heat treatment, in the presence of an aqueous solution containing at least one hydrotropic agent, at a concentration at least equal to the minimum hydrotropic concentration, followed by an operation to recover the extract, **characterized in that** the hydrotropic agent is an alkyl-(poly)glycoside of general formula Alk-O-Zp, wherein:
• Alk denotes a saturated or unsaturated, linear or branched hydrophobic aliphatic hydrocarbon fragment having 3 to 7 carbon atoms, and
• Z is a hydrophilic glycoside group such as glucose, xylose and arabinose, and
• 1<p<5

2. The method according to claim 1, **characterized in that** said heat treatment is conducted at temperatures between 20°C and 200°C, preferably between 60°C and 120°C.

3. The method according to one of claims 1 or 2, **characterized in that** the plants subjected to thermomechanical treatment in the presence of an aqueous solution of a hydrotropic agent are composed in full or part of the above-ground parts and/or below-ground parts of dry plants or of fresh plants that are whole, fragmented or ground.

4. The method according to claim 3 wherein the plant parts are represented by the above-ground parts such as the stems, branches, leaves, fruit, seeds and/or flowers; and/or the below-ground parts such as rhizomes, roots and/or bulbs.

5. The method according to one of claims 1 to 4, **characterized in that** the dry or fresh plants subjected to thermomechanical treatment in the presence of an aqueous solution of a hydrotropic agent are selected from the fruit of *Physalis peruviana,* seeds of *Embelia ribes,* leaves of *Myrtus communis,* leaves of *Eucalyptus globulus,* pericarps of *Garcinia mangostana,* female inflorescences of *Humulus lupulus,* bark of *Cinchona* sp., above-ground parts of *Urtica dioica,* above-ground parts of *Helichrysum* spp., fruit of *Vanilla* spp., rhizomes of *Zingiber officinale,* rhizomes of *Curcuma* spp., rhizomes of *Piper methysticum,* leaves of *Piper* spp., fruit and leaves of *Olea europaea.*

6. The method according to one of the preceding claims, **characterized in that** the plants undergo pretreatment before extrusion, treatment by ultrasound, microwave, enzymatic digestion, maceration in the hydrotropic solution, preparation of the plant such as cryogenic grinding, fragmentation, grinding, in particular maceration in the hydrotropic solution.

7. The method according to one of the preceding claims, **characterized in that** the recovered extract is subjected to a subsequent step of clarification and/or filtration.

8. The method according to claims 1 to 6 **characterized in that** the recovered extract is diluted, concentrated, dried or stored as such with the addition of suitable preserving agent.

9. The method according to claims 1 to 6 **characterized in that** the recovered extract is diluted in a sufficient volume of water to recover a dry extract enriched with lipophilic compounds.

10. The method according to one of the preceding claims, **characterized in that** extrusion is implemented in a twin-screw extruder.

11. The method according to claim 10, **characterized in that** the twin-screw extruder comprises a first zone with co-rotating and co-penetrating twin-screws, where trituration of said plants takes place.

12. The method according to one of claims 10 and 11, **characterized in that** the twin-screw extruder comprises a second twin-screw zone where solid/liquid separation takes place.

13. The method according to one of claims 1 to 12, **characterized in that** said extruder comprises at least one barrel and preferably several successive adjacent barrels.
